# NOUVEAU FASCICULE DE BREVET EUROPEEN

(11) **EP 0 092 287 B2**
(45) Date de publication et mention de la décision concernant l'opposition: **23.11.1994**
(45) Mention de la délivrance du brevet: 25.03.1987
(21) Numéro de dépôt: 83200547.4
(22) Date de dépôt: 18.04.1983
(51) Int. Cl.: A61K 31/205, C07C 323/02, C07C 229/04

(54) **Sels d'acétylcystéine, leur préparation et leur utilisation**
Acetylcystein-Salze, ihre Herstellung und Verwendung
Acetylcysteine salts, their preparation and use

(30) Priorité: 19.04.1982 LU 84095
(43) Date de publication de la demande: 26.10.1983
(73) Titulaire: "PHARLYSE", Société Anonyme, Luxembourg (LU)
(72) Inventeur: Deboeck, Arthur Marie, B-1540 Herne (BE)
(74) Mandataire: Schmitz, Yvon

(56) Documents cités:
- JP-A-81 155 298
- US-A- 3 091 569
- US-A- 4 206 137
- Chemical Abstracts vol. 96, no. 22, 31 mai 1982, Columbus, Ohio, USA, page 400, colonne 2, abstract no. 187088g
- Martindale: The Extra Pharmacopoeia (1982) 28th ed., pages 644-645
- US Pharmacopoeia XXI (1985), pages 7, 19, 20
- Remington's 17th ed. Pharm. Sciences (1985) pages 867-868, Lysomucil

## Description

La présente invention est relative à des sels pharmaceutiquement utilisables, solubles dans l'eau, de la N-acétyl-L-cysteine.

N-acétyl-L-cystéine ou acétylcystéine est un médicament bien connu, particulièrement par ses propriétés mucolytiques et comme antidote lors d'intoxication aigué au paracétamol. Il présente néanmoins l'inconvénient d'être extrêmement peu soluble dans l'eau, ce qui gêne son utilisation dans la plupart des formes pharmaceutiques. Des sels de métaux alcalins d'acétylcystéine peuvert être préparés à cet effet mais ils ont le désavantage d'apporter avec eux des ions alcalins, notamment du sodium, qui sont souvent contre-indiqués.

On notera qu'il est connu suivant le document JP-A-81-155 298 de préparer des sels d'acides aminés dicarboxyliques ou d'acides aminés contenant du soufre, N-acylés au départ d'acides aminés basiques. mais que ces sels sont utilisés dans la préparation de shampooings, dans le but d'améliorer l'état des cheveux, et que leur application est donc tout à fait différente de celle des sels de l'invention, et ne laissent en aucun cas supposer qu'ils pourraient avoir une activité mucolytique ou être utilisés comme antidotes lors d'intoxication aigué au paracétamol. Un exemple de ces sels utilisables dans la préparation de shampooings est l'aspartate d'arginine. On se référera également, à cet effet, aux Chemical Abstracts vol. 26, n _{°} 22, 31 mai 1982, Columbus, Ohio, USA page 400, colonne 2, résumé n 187088 g ainsi qu'au Chemical Substance Index, vol. 96, 1982, page 216 Cs, colonne 2, lignes 46-47.

La présente invention a pour but de mettre au point un sel d'acétylcystéine pour l'utilisation comme medicament mucolytique ou comme antidote lors d'intoxication aigué au paracétamol, fortement soluble dans l'eau ne présentant pas les inconvénients des sels de métaux alcalins. A cet effet, suivant l'invention, le sel est constitué par le produit de réaction de l'acétylcystéine et au moins d'un acide aminé basique choisi dans le groupe forme par l'arginine, la lysine, l'histidine et l'ornithi- ne.

Avantageusement, le sel contient environ 1 à 5 moles, et de préférence environ 1 mole de ces acides aminés basiques par mole d'acétylcystéine. Le ou les acides amines basiques utilisés peuvent être naturels ou non. Les acides aminés basiques selon la présente inventon renferment un centre asymétrique et peuvent donc exister sous les formes isomères optiquement actives. Il est bien entendu que l'invention couvre les deux formes épi- mères, a savoir les formes lévogyre et dextrogyre, ainsi que leur mélange. Des exemples d'acides aminés basiques lévogyres et dextrogyres sont les D- et L-lysines et les D- et L-arginines.

Selon une première façon de preparer ces sels d'acetylcysteine, l'acétylcystéine est mise en réaction avec une solution aqueuse contenant le ou les acides aminés basiques. Après réaction, l'eau est ensuite séparée du mélange réactionnel ainsi obtenu par des méthodes de séparation appropriées, telles que par évaporation ou lyophilisation.

Une autre façon de procéder consiste à mettre en réaction une solution ou dispersion aqueuse ou hydroorganique contenant le ou les acides aminés basiques avec une solution ou dispersion organique contenant l'acétylcystéine, avec une solution aqueuse contenant l'acétylcystéine ou bien avec l'acétylcystéine telle quelle, et à séparer le solvant du mélange réactionnel ainsi obtenu, par des méthodes de séparation appropriées, telles que par filtration, lyophilisation ou évaporation. Des exemples de solvants organiques utilisés pour dissoudre les acides aminés et l'acétylcystéine sont les solvants organiques polaires, tels que les alcools, les glycols, les polyglycols, les cétones, le diméthylfor- mamide et le diméthylsulfoxyde. On peut également utiliser des mélanges de tels solvants.

Une troisième façon de procéder consiste à mettre en contact un ou des sels de l'acide aminé ou des acides aminés basiques en solution aqueuse, organique ou hydroorganique avec de l'acétylcystéine en solution aqueuse organique ou hydroorganique, et à séparer le solvant du milieu réactionnel par des méthodes de séparations appropriées, telles que par évaporation, lyophilisation ou filtration. Un exemple de sel d'acide aminé est le carbonate de lysine. Des exemples de solvants de précipitation sont les acétates d'alkyle, l'éther sulfurique, le dioxane, le tétrahydrofuranne, les cétones, les alcools et leurs mélanges.

Une autre façon de procéder consiste à mélanger intimement à sec les réactifs solides, c'est-à-dire le ou les acides aminés basiques ou leurs sels avec l'acétylcystéine. La poudre ainsi obtenue est immédiatement utilisable pour la réalisation de préparations à dissoudre au moment de l'emploi. Dans le cas où le carbonate d'un acide aminé ou d'un mélange d'acides aminés est utilisé, la poudre obtenue, après adjonction d'un acide ou d'un mélange d'acides pharmaceutiquement acceptables, permet l'obtention de préparations effervescentes.

Dans les quatre façons de procéder, le traitement susdit est effectué à une température de l'ordre de - 5 à 100 _{°} C, et de préférence à une température voisine de 20 ° C.

L'acétylcystéine est un thioacide solide peu soluble dans l'eau. Ainsi qu'on l'a déjà précisé, les sels se forment aisément avec les acides aminés basiques, a savoir l'arginine, la lysine, l'histidine et l'ornithine.

Les sels de l'acétylcystéine parfaitement solubles dans l'eau requièrent de 0,5 à 5 molécules d'acide aminé et de préférence 1 molécule d'acide aminé par molécule d'acétylcystéine.

En fait, les sels d'acétylcystéine de l'invention répondent à la formule suivante : 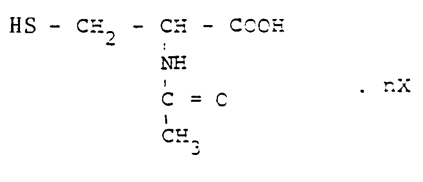 dans laquelle n, représentant le nombre de molécules d'acide aminé par rapport à 1 molécule d'acétylcystéine, est compris entre 1 et 5, et de préférence compris entre 1 et 2, et X représente le ou les acides aminés.

Les sels de l'acétylcystéine hydrosolubles de la présente invention peuvent donc être obtenus en utilisant des méthodes connues de préparation de sels, et, en particulier, par la mise en solution ou suspension aqueuse, hydroorganique ou organique d'un ou de plusieurs acides aminés, ou par la mise en solution ou suspension aqueuse, hydroorganique ou organique d'un ou de plusieurs sels d'acides aminés, à laquelle, tout en maintenant la température comprise entre 0 _{°} C et 100 _{°} C et de préférence aux alentours de 20 _{°} C, on ajoute, sous agitation, par petites portions la quantité d'acétylcystéine éventuellement sous forme de solution organique, hydroorganique ou aqueuse. On notera, à cet effet. que l'on peut inverser l'ordre d'addition des réactifs mis en présence. Lorsque la solution est devenue limpide, on élimine le solvant du mélange réactionnel par des méthodes de séparation appropriées quelconques, telles que, par exemple, par lyophilisation (solution aqueuse) ou par chauffage modéré sous vide. On obtient ainsi une poudre soluble dans l'eau qui peut être utilisée pour la préparation de formes solides (comprimés, suppositoires, tablettes, granulés, dragées) et de formes injectables. Il va de soi que les solutions de sels d'acétylcystéine préparées comme expliqué ci-dessus, peuvent être utilisées immédiatement sous forme injectable sans être lyophilisées au préalable, et pour autant que leur force ionique soit acceptable ou rendue telle. Ci-dessous sont rassemblés quelques exemples, non limitatifs, de préparation des composés suivant l'invention.

### Exemple 1

A une solution aqueuse contenant 6,75 g de lysine base dans 100 ml, on ajoute progressivement et sous agitation, 7.58 g d'acétylcystéine. Le pH est ajusté à 6,5 par addition soit d'acétylcystéine soit de lysine base. La solution ainsi obtenue est congelée et lyophilisée. On obtient 14,3 g d'acétylcystéinate de lysine instantanément soluble dans l'eau et présentant les caractéristiques suivantes :
P. F. 290-295 _{°} C, décomposition
Solubilité : supérieure à 30 %.

En procédant de la même façon avec l'arginine, l'histidine ou l'ornithine, on obtient les sels correspondants qui sont tous solubles dans l'eau.

### Exemple 2

A une solution de 14,6 g de lysine base dans 10 ml d'eau et 50 ml de méthanol, on ajoute par petites portions 16,3 g d'acétylcystéine. Après environ 1 heure de réaction, la solution est évaporée à sec. La poudre blanche ainsi obtenue est triturée avec 100 ml d'éthanol et filtrée. Après séchage à 60 _{°} C, on obtient environ 28 g (90%) d'acétylcys- téinate de lysine instantanément soluble dans l'eau.

### Exemple 3

A une suspension de 14,6 g de lysine base dans 150 ml d'éthanol, on ajoute sous agitation 16.3 g d'acétylcystéine. Le produit cristallise au fur et à mesure de sa formation. Après 2 heures de réaction, le milieu est filtré, séché à 60 _{°}C. On obtient environ 28 g (90,0 %) d'acétylcystéinate de lysine.

### Exemple 4

A une solution de 16,8 g de carbonate de lysine dans un mélange de 20 ml d'eau et 50 ml d'éthanol. On ajoute par petites portions 16,3 g d'acétylcystéine. Après 1 heure de réaction, la solution limpide est évaporée sous vide. L'huile obtenue après évaporation est additionnée de 200 ml d'éthanol et le sel cristallise. Après filtration et séchage, on obtient 27,9 g (90,0 %). L'acétylcystéi- nate de lysine obtenu sous forme de poudre blanche est instantanément soluble dans l'eau.

Les sels d'acétylcystéine solubles dans l'eau de la présente invention exercent une activité mucolytique remarquable et sont également d'excellents antidotes lors d'intoxication aiguë au paracétamol. Les sels à base d'acétylcystéine de l'invention peuvent être administrés en association avec divers excipients pharmaceutiques, tels que des diluants, des gélifiants, des agents conservateurs, des émulsionnants, des édulcorants et aromatisants, etc..., et cela par voie orale, parentérale ou rectale.

Pour une administration orale, on utilisera des dragées, granulés, pastilles (pellets), tablettes, capsules, comprimés, solutions, sirops, émulsions contenant des additifs ou excipients classiques en pharmacie galénique. Ces formes galéniques peuvent libérer le principe de façon normale ou programmée dans le temps.

Les sels de la présente invention peuvent également être administrés sous forme d'aérosols ou de nébulisats, l'acétylcystéinate étant soit dissous dans un solvant approprié soit sous forme de poudre.

Pour l'administration parentérale, on utilisera tout véhicule approprié, comme, par exemple, de l'eau stérile, une huile d'arachide ou de l'oléate d'éthyle.

Pour l'administration rectale, on utilisera des suppositoires, des capsules rectales, des solutions ou des gelées.

Le composé actif peut être administré seul ou en combinaison avec d'autres produits actifs ayant une activité similaire ou différente.

Les doses recommandées pour l'administration par la voie orale et rectale sont, par exemple, de 100 mg à 20 g, avantageusement de 500 mg à 10 g par jour.

## Revendications

1. Sel de N-acétyl-L-cysteine hydrosoluble pour l'utilisation comme medicament mucolytique ou comme antidote lors d'intoxication aiguë au paracétamol, caractérisé en ce qu'il est constitué par le produit de reaction de la N-acétyl-L-cystéine et au moins d'un acide aminé basique choisi dans le groupe formé par l'arginine, la lysine, l'histidine et l'ornithine.

2. Sel suivant la revendication 1, caractérisé en ce que l'acide aminé basique est un acide aminé lévogyre, dextrogyre ou un mélange de tels acides.

3. Sel suivant l'un au l'autre des revendications 1 et 2 caractérisé en ce qu'il contient environ 1 à 5 moles d'acide aminé basique par mole de N-acétyl-L-cystéine.

4. Sel suivant la revendication 3, caractérisé en ce qu'il contient environ 1 mole d'acide aminé basique par mole de N-acétyl-L-cysteine.

5. Compositions pharmaceutiques comprenant, comme produit actif, au moins un sel de N-acétyl-L-cysteine hydrosoluble suivant l'une quelconque des revendications 1 à 4, associé à au moins un excipient approprié et/ou à au moins un autre agent thérapeutique.

## Claims

1. A water-soluble N-acetyl-L-cysteine salt for use as a mucolytic drug or as antidote against acute intoxication with paracetamol, characterized in that it is formed by the reaction product between N-acetyl-L-cysteine and at least one basic amino-acid selected from the group comprising arginine, lysine, histidine and or- nithine.

2. A salt as claimed in claim 1, characterized in that the basic amino-acid is a levorotary amino-acid, a dextrorotary amino-acid or a mixture of said acids.

3. A salt as claimed in any of claims 1 and 2, characterized in that it contains about 1 to 5 moles of basic amino-acid per mole of N-acetyl-L-cysteine.

4. A salt as claimed in claim 3, characterized in that it contains about 1 mole of basic amino-acid per mole of N-acetyl-L-cysteine.

5. A pharmaceutical composition, comprising as active product at least one water-soluble N-acetyl-L-cysteine salt according to any of claims 1 to 4, in association with at least a suitable excipient and/or at least another therapeutical agent.

## Patentansprüche

1. Wasserlösliches N-Acetyl-L-Cystein-Salz zur Verwendung als schleimlösendes Mittel oder als Gegenmittel bei einer schweren Paracetamol-Vergiftung, dadurch gekennzeichnet, daß es aus dem Reaktionsprodukt von N-Acetyl-L-Cystein und mindestens einer basischen Aminosäure besteht, die aus der von Arginin, Lysin, Histidin, und Ornithin gebildeten Gruppe ausgewählt wird.

2. Salz nach Anspruch 1, dadurch gekennzeichent, daß die basische Aminosäure eine linksdrehende oder eine rechtsdrehende Aminosäure ist oder aus einer Mischung solcher Aminosäuren besteht.

3. Salz nach dem einen oder anderen der Ansprüche 1 und 2, dadurch gekennzeichnet, daß es etwa 1 bis 5 Mol basische Aminosäure pro Mol N-Acetyl-L-Cystein enthält.

4. Salz nach Anspruch 3, dadurch gekennzeichnet, daß es etwa 1 Mol basische Aminosäure pro Mol N-Acetyl-L-Cystein enthält.

5. Pharmazeutische Zubereitung, die als Wirkstoff wenigstens ein wasserlösliches N-Acetyl-L-Cystein-Salz nach einem der Ansprüche 1 bis 4 enthält, in Verbindung mit wenigstens einem geeigneten Hilfsstoff und/oder Wenigstens einem anderen therapeutischen Wirkstoff.
